Europäisches Patentamt

European Patent Office

Office européen des brevets

(19)

(11) **EP 0 906 084 B1**

(12) **FASCICULE DE BREVET EUROPEEN**

(45) Date de publication et mention
de la délivrance du brevet:
**16.10.2002 Bulletin 2002/42**

(21) Numéro de dépôt: **97920803.0**

(22) Date de dépôt: **22.04.1997**

(51) Int Cl.7: **A61K 7/16**, C09C 3/06

(86) Numéro de dépôt international:
**PCT/FR97/00721**

(87) Numéro de publication internationale:
**WO 97/039728 (30.10.1997 Gazette 1997/46)**

(54) **COMPOSITION DENTIFRICE COMPRENANT UN ABRASIF OU ADDITIF A BASE DE SILICE ET DE CARBONATE DE CALCIUM, COMPATIBLE AVEC LE FLUOR**

ZAHNPASTA DIE EIN FLUORIDVERTRÄGLICHES SCHLEIFMITTEL ODER ZUSATZSTOFF AUF BASIS VON KIESELSÄURE UND KALZIUMCARBONAT ENTHÄLT

TOOTHPASTE COMPOSITION COMPRISING A SILICA AND CALCIUM CARBONATE BASED ABRASIVE OR ADDITIVE, COMPATIBLE WITH FLUORINE

(84) Etats contractants désignés:
**AT BE CH DE DK ES FI FR GB GR IE IT LI LU MC NL PT SE**
Etats d'extension désignés:
**RO SI**

(30) Priorité: **22.04.1996 FR 9605135**

(43) Date de publication de la demande:
**07.04.1999 Bulletin 1999/14**

(73) Titulaire: **RHODIA CHIMIE**
**92408 Courbevoie Cédex (FR)**

(72) Inventeurs:
• **AMICHE, Frédéric**
**F-92420 Vaucresson (FR)**

• **DROMARD, Adrien**
**F-69006 Lyon (FR)**

(74) Mandataire: **Fabre, Madeleine-France et al**
**Rhodia Services,**
**Direction de la Propriété Industrielle,**
**40, rue de la Haie-Coq**
**93306 Aubervilliers Cedex (FR)**

(56) Documents cités:
**EP-A- 0 219 483          WO-A-93/22386**
**US-A- 2 885 366          US-A- 4 420 312**

• **DATABASE WPI Section Ch, Week 9546 Derwent Publications Ltd., London, GB; Class B07, AN 95-355057 XP002022183 & JP 07 242 409 A (NIPPON INSULATION KK) , 19 Septembre 1995**

**Description**

La présente invention a pour objet des compositions dentifrices contenant, comme agent abrasif ou additif compatible avec le fluor, des particules comprenant un coeur de carbonate de calcium et une écorce de silice active de précipitation contenant éventuellement à l'état adsorbé un agent stabilisant des ions $Ca^{2+}$ ; elle a également pour objet l'utilisation, comme agent abrasif ou additif pour compositions dentifrices, de particules composites comprenant un coeur de carbonate de calcium et une écorce de silice active de précipitation contenant éventuellement à l'état adsorbé un agent stabilisant des ions $Ca^{2+}$. Elle a en outre pour objet, à titre de produit industriel nouveau, des particules composites constituées d'un coeur de carbonate de calcium et d'une écorce de silice active de précipitation contenant à l'état adsorbé un agent stabilisant des ions $Ca^{2+}$.

La silice et le carbonate de calcium sont couramment utilisés dans les compositions dentifrices comme agents abrasifs en aidant par leur action mécanique à l'élimination de la plaque dentaire. La silice peut également jouer le rôle d'épaississant pour conférer des propriétés rhéologiques déterminées au dentifrice.

Les compositions dentifrices contiennent divers additifs autres, notamment des additifs pour la prévention des caries, en particulier des fluorures.

La présence de fluorures pose le problème de leur compatibilité avec les abrasifs à base de calcium (carbonate de calcium par exemple), qui, de par leurs propriétés de surface, peuvent limiter la disponibilité des fluorures à exercer leur effet thérapeutique.

Des silices de précipitation traitées par un sel de calcium hydrosoluble ont déjà été proposées comme agent abrasif dans les pâtes dentifrices (US-A-4,420,312).

Des silices amorphes obtenues par carbonisation des particules de silicate de calcium en présence d'eau ou de silicate de métal alcalin puis séparation, sont également connues comme additif pour pâtes dentifrice (JP 07 242 409; DATABASE WPI-AN 95-355057).

Il est connu de préparer des particules comprenant une écorce de silice active dense et un coeur de carbonate de calcium selon les procédés classiques par précipitation lente sur le coeur de carbonate de calcium, de silice active à partir d'une solution aqueuse de silicate de métal alcalin avec réglage du pH à l'aide d'un acide (US-A-2,885,366).

La demanderesse a trouvé que les particules constituées d'une écorce de silice et d'un coeur de carbonate de calcium sont compatibles avec le fluor et sont donc particulièrement intéressantes dans les compositions dentifrices.

La présente invention a donc pour objet une composition dentifrice comprenant au moins un agent pour la prévention des caries, à base d'un composé fluoré en une quantité correspondant à une concentration de 0,005 à 2%, de préférence de 0,1 à 1% en poids de fluor dans ladite composition, et au moins une silice, composition caractérisée en ce que ladite silice représente de 5 à 40% , de préférence de 5 à 35% du poids de ladite composition et est constituée de particules comprenant une écorce de silice active et un coeur de carbonate de calcium susceptibles d'être obtenues par précipitation sur du carbonate de calcium, de silice active à partir d'une solution aqueuse de silicate de métal alcalin M, de rapport $SiO_2/M_2O$ de 2 au moins, de préférence de 2,5 à 4, avec réglage du pH à l'aide d'un agent acidifiant, séparation de la bouillie de silice formée et séchage de la suspension de silice récupérée.

Lesdites particules de silice peuvent présenter une écorce de silice active (hydroxylée) d'épaisseur de l'ordre de 2 à 200 nm, de préférence de 5 à 50 nm, pour une taille de coeur de carbonate de calcium de l'ordre de 20nm à 30 $\mu$m, de préférence de l'ordre de 50 nm à 20$\mu$m.
Leur surface spécifique BET est généralement de l'ordre de 1 à 100 $m^2/g$, de préférence de l'ordre de 1 à 40 $m^2/g$. Leur abrasivité RDA peut être de l'ordre de 30 à 250, de préférence de l'ordre de 40 à 200.

La surface spécifique BET est déterminée selon la méthode de BRUNAUER - EMET - TELLER décrite dans "The journal of the American Chemical Society", Vol. 60, page 309, février 1938 et correspondant à la norme NFT 45007 (novembre 1987).

L'abrasivité RDA ("Radioactive Dentine Abrasion") est mesurée selon la méthode décrite par J.J. HEFFERREN dans "Journal of Dental Research", vol. 55(4), page 563, 1976.

Quant au coeur de carbonate de calcium il peut s'agir de carbonate précipité (aragonite, calcite), de carbonates naturels broyés ...
Celui-ci présente de préférence un diamètre médian de l'ordre de 50nm à 20$\mu$m.

Selon un procédé de préparation particulièrement performant des particules de silice selon l'invention, l'opération de formation de bouillie de silice par précipitation est réalisée selon les étapes suivantes :

- une première étape consistant à mettre en oeuvre un pied de cuve initial de pH de 8 à 10, comprenant de l'eau, du carbonate de calcium, un sel électrolyte du groupe des métaux alcalins, la quantité d'électrolyte présente étant d'au moins 0,4 mole, de préférence de 0,4 à 1,5 mole d'ion métal alcalin par litre de pied de cuve, éventuellement un agent basique ou tampon, à une température de 80 à 98°C ;
- une deuxième étape consistant à introduire dans ledit pied de cuve, le silicate de métal alcalin sous forme d'une solution aqueuse contenant au moins 100 grammes de $SiO_2$ / litre , de préférence de 100 à 330 grammes de $SiO_2$

/ litre, et l'agent acidifiant, dans des conditions telles que la cinétique C de formation de silice active, exprimée en grammes de silice / heure / gramme de carbonate de calcium, corresponde à une valeur

$$C \geq 3 \, (A/200) \, 2^n,$$

de préférence $C \geq 4 \, (A/200) \, 2^n$
et tout particulièrement $C \geq 6 \, (A/200) \, 2^n$

. $\underline{n}$ étant égal à (T-90) / 10
. $\underline{A}$ représentant la surface spécifique, exprimée en $m^2/g$, du carbonate à enrober
. et T la température en °C,

le milieu réactionnel présentant un pH sensiblement constant de 8 à 10 et étant maintenu à une température de 80 à 98°C,
et ce jusqu'à ce que la quantité désirée de silice soit formée.

Le choix du silicate et de l'agent acidifiant se fait d'une manière bien connue en soi. Le silicate de métal alcalin est avantageusement un silicate de sodium ou de potassium.

On utilise généralement comme agent acidifiant un acide minéral tel que l'acide sulfurique, l'acide nitrique, l'acide chlorhydrique ou un acide organique comme l'acide acétique, l'acide formique ou l'acide carbonique. D'une manière préférentielle, il s'agit de l'acide sulfurique. Ce dernier peut être mis en oeuvre sous forme dilué ou concentré, de préférence sous forme d'une solution aqueuse présentant une concentration de l'ordre de 60 à 400 g/l. S'il s'agit d'acide carbonique, celui-ci peut être introduit sous forme gazeuse.

La première étape consiste à préparer le pied de cuve initial.

Le carbonate de calcium est de préférence introduit sous forme d'une dispersion aqueuse.

La quantité de carbonate de calcium pouvant être mise en oeuvre est telle que le pied de cuve formé contienne de 10% à 50% de son poids de carbonate de calcium.

Parmi les électrolytes on peut citer notamment le sel du métal de silicate de départ et de l'agent acidifiant ; de préférence il s'agit de sulfate de sodium ; toutefois du chlorure, nitrate ou hydrogénocarbonate de sodium peut être préféré si la présence d'ions sulfates résiduels n'est pas désirée.

Un agent basique ou tampon peut être mis en oeuvre dans le pied de cuve initial pour assurer un pH dudit pied de cuve de 8 à 10.
Cet agent basique ou tampon peut être choisi parmi les hydroxydes de métaux alcalins comme l'hydroxyde de sodium, les silicates de métaux alcalins en solution, les phosphates de métaux alcalins, les hydrogénocarbonates de métaux alcalins ...

Le pied de cuve obtenu est porté à une température de 80 à 98°C.

La deuxième étape consiste à ajouter au pied de cuve maintenu sous agitation, la solution de silicate et l'agent acidifiant simultanément.

Les quantités respectives de silicate de métal alcalin et d'agent acidifiant sont choisies de manière à obtenir la cinétique C de formation de silice active mentionnée ci-dessus et de manière à maintenir le pH du milieu réactionnel à une valeur sensiblement constante de 8 à 10 pendant toute l'introduction des deux réactifs.
Ces deux solutions sont introduites en maintenant le milieu à une température de 80 à 98°C.
On arrête l'introduction de la solution de silicate lorsque la quantité désirée de silice est formée. La quantité minimum recherchée de silice est celle correspondant à un dépot de 1 à 150 parties en poids de $SiO_2$ pour 100 parties en poids de carbonate de calcium.

Cette deuxième étape dure généralement de 30 minutes à 2 heures.

Le milieu obtenu en fin de deuxième étape, après arrêt de l'introduction des réactifs, est éventuellement laissé mûrir pendant environ 10 à 30 minutes dans les mêmes conditions de température.

On obtient à l'issue des opérations ci-dessus décrites, une bouillie de particules comprenant de la silice active déposée sur du carbonate de calcium, bouillie qui est ensuite séparée (séparation liquide-solide) ; cette opération consiste généralement en une filtration (par exemple décantation, utilisation d'un filtre rotatif sous vide), suivie d'un lavage à l'eau.

La suspension ainsi récupérée (gâteau de filtration) est ensuite séchée (étuve, four, atomisation).
Les composés fluorés sont en particulier les sels de l'acide monofluorophosphorique, notamment ceux de sodium, potassium, lithium, calcium, aluminium et ammonium, les fluorures de métaux alcalins, de sodium notamment.

Une variante de la composition dentifrice faisant l'objet de l'invention consiste en ce que l'écorce de silice des particules comprenant une écorce de silice active de précipitation et un coeur de carbonate de calcium selon l'invention, contient à l'état adsorbé au moins un agent stabilisant des ions $Ca^{2+}$.

Parmi les agents stabilisants on peut citer des dérivés hydrosolubles du phosphore comme les phosphates de métaux alcalins (pyrophosphates, orthophosphates, tripolyphosphates, hexametaphosphates de sodium, potassium, lithium).

La quantité dudit agent stabilisant peut représenter jusqu'à 2% de la masse des particules comprenant une écorce de silice active dense et un coeur de carbonate de calcium, exprimée en sec.

Lesdites particules peuvent être obtenues par traitement de l'écorce de silice par une solution aqueuse dudit agent stabilisant, traitement réalisé par addition de ladite solution soit à la bouillie de silice formée à la fin de l'étape de précipitation, avant filtration, soit à la suspension de silice (gâteau de filtration) obtenue après filtration, avant séchage.

Un autre mode de réalisation consiste à introduire directement ledit agent stabilisant dans la composition dentifrice elle-même.

Les compositions dentifrices contenant des particules comprenant un coeur de carbonate de calcium et une écorce de silice active de précipitation contenant à l'état adsorbé au moins un agent stabilisant des ions $Ca^{2+}$, peuvent en particulier contenir, comme composé fluoré, un fluorure de métal alcalin, comme le fluorure de sodium.

La composition dentifrice faisant l'objet de l'invention, peut contenir en outre :

- des agents tensio-actifs anioniques, non-ioniques, amphotères ou zwitterioniques, à raison d'environ 0,1 à 10% , de préférence d'environ 1 à 5% du poids de ladite composition ; on peut citer, à titre d'exemple

  * des tensio-actifs anioniques comme les sels de sodium, de magnésium, d'ammonium, d'éthanolamine, des

    . alkyl sulfates en $C_8$-$C_{18}$ pouvant éventuellement contenir jusqu'à 10 motifs oxyéthylène et ou oxypropylène (laurylsulfate de sodium notamment)
    . alkyl sulfoacétates en $C_8$-$C_{18}$ (laurylsulfoacétate de sodium notamment)
    . alkyl sulfosuccinates en $C_8$-$C_{18}$ (dioctylsulfosuccinate de sodium notamment)
    . alkyl sarcosinates en $C_8$-$C_{18}$ (laurylsarcosinate de sodium notamment)
    . alkyl phosphates en $C_8$-$C_{18}$ pouvant éventuellement contenir jusqu'à 10 motifs oxyéthylène et ou oxypropylène
    . alkyl ether carboxylates en $C_8$-$C_{18}$ contenant jusqu'à 10 motifs oxyéthylène et ou oxypropylène
    . les monoglycérides sulfatés ...

  * des agents tensio-actifs non-ioniques comme les esters gras de sorbitan éventuellement polyéthoxylés, les acides gras éthoxylés, les esters de polyéthylèneglycol....
  * agents tensio-actifs amphotères comme les bétaines, sulfobétaines

- de l'eau à raison d'environ 5 à 50%, de préférence environ 10 à 40% du poids de ladite composition
- des agents humectants, à raison d'environ 10 à 85%, de préférence de 10 à 70% du poids de ladite composition, humectants comme le glycérol, le sorbitol, les polyéthylèneglycols, le lactilol, le xylitol ...
- des agents épaississants comme certaines silice utilisées à cet effet (TIXOSIL 43® commercialisée par RHONE-POULENC ...) à raison de 5 à 15% en poids et/ou des polymères utilisés seuls ou en association comme la gomme Xanthane, la gomme guar, les dérivés de la cellulose (Carboxyméthylcellulose, hydroxyéthylcellulose, hydroxypropylcellulose, hydroxypropylméthylcellulose..... ), des polyacrylates réticulés comme les CARBOPOL® distribués par GOODRICH, des alginates ou des carraghénannes, de la VISCARIN® ... , à raison de 0,1 à 5% en poids
- des agents stabilisants des ions $Ca^{2+}$ tels que des dérivés hydrosolubles du phosphore comme les phosphates de métaux alcalins (pyrophosphates, orthophosphates, tripolyphosphates, hexametaphosphates de sodium, potassium, lithium)
- d'autres ingrédients, tels que

  * des abrasifs polissants autres, tels que la silice, le carbonate de calcium précipité, le carbonate de magnésium, les phosphates de calcium, les oxydes de titane, zinc, étain, le talc, le kaolin ...
  * des agents thérapeutiques bactéricides, anti-microbiens, anti-plaque, comme le citrate de zinc, les polyphosphates, les guanidines, les bis-biguanides ou autre composé organique thérapeutique cationique
  * des agents arômatisants, (essence d'anis, de badiane, de menthe, de genièvre, cannelle, girofle, rosé, ), des édulcorants, des colorants (chlorophylle) , des conservateurs ...

La composition dentifrice faisant l'objet de l'invention, peut se présenter sous différentes formes (pâtes, gels, crèmes), préparées à l'aide des procédés conventionnels.

La compatibilité des particules comprenant une écorce de silice et un coeur de carbonate de calcium (particules dénommées "abrasif" dans les tests), avec le monofluorophosphate de sodium (MFP) d'une part, et avec le fluorure de sodium (NaF) d'autre part, a été mésurée selon les tests suivants :

Mesure de la comoatibilité MFP

On prépare une suspension à 20% en poids d'abrasif, par mise en contact à 60°C de 10g d'abrasif dans 40g d'une solution mère à 625 ppm de MFP dans un solvant choisi parmi l'eau, un mélange 50/50 sorbitol/eau ou un mélange 35/65 glycérol/eau.

La suspension, ainsi qu'un échantillon témoin comportant la solution mère sans abrasif, sont maintenues sous agitation magnétique à l'étuve 37°C pendant 4 semaines.

L'échantillon témoin ainsi que la suspension, sont ramenés à température ambiante et centrifugés.

Le surnageant est prélevé puis filtré, de même que la solution témoin.

Le dosage par ionométrie du fluor total soluble dans le surnageant nécessite au préalable un traitement d'hydrolyse en milieu acide, qui libère les ions fluors présents sous forme de complexe dans le MFP.

L'hydrolyse est réalisée par mise en présence de 25ml de surnageant avec 5ml d'acide sulfurique 5N et 20ml d'eau désionisée, et mise à l'étuve à 60°C pendant au moins 5 heures.

La compatibilité de l'abrasif, exprimé en %, est définie par le rapport $(C/C_0) \times 100$,

C représentant la concentration en fluor du surnageant testé (après hydrolyse) et $C_0$ la concentration en fluor du témoin (après hydrolyse).

La concentration en ion fluor du surnageant a été en outre mesurée avant hydrolyse acide, ce afin d'avoir accés aux concentrations en fluor résultant de l'hydrolyse du MFP.

Dosage du fluor total soluble dans les pâtes dentifrices contenant du MFP

10g de pâte dentifrice de composition suivante :

| composition | % en poids |
|---|---|
| carboxyméthylcellulose | 0,8 |
| sorbitol | 18 |
| monofluorophosphate de sodium | 0,8 |
| saccharinate de sodium (édulcorant) | 0,2 |
| benzoate de sodium (conservateur) | 0,3 |
| abrasif testé | 40 |
| TIXOSIL 43 (silice épaississante) | 5 |
| laurylsulfate de sodium (solution aqueuse à 30%) | 4 |
| arôme | 0,8 |
| eau désionisée | 30,1 |

sont mis en suspension dans 90g d'eau, pendant 5 mois à température ambiante.

La suspension est ensuite centrifugée et le surnageant filtré.

Le dosage par ionométrie du fluor total soluble dans le surnageant nécessite au préalable un traitement d'hydrolyse en milieu acide, qui libère les ions fluors présents sous forme de complexe dans le MFP.

L'hydrolyse est réalisée par mise en présence de 25ml de surnageant avec 5ml d'acide sulfurique 5N et 20ml d'eau désionisée, et mise à l'étuve à 60°C pendant au moins 5 heures.

La concentration en fluor du surnageant testé est mesurée après hydrolyse, de même qu'avant hydrolyse.

Mesure de la compatibilité vis-vis du fluorure de sodium NaF

Le principe de la mesure consiste à laisser en contact l'abrasif à tester avec une solution de fluorure de sodium de concentration connue, pendant 10 jours à 37°C.

La quantité de fluorure présent dans le milieu liquide obtenu par centrifugation, est mesuré par ionométrie.

- Mode opératoire -

La silice à tester est mise en suspension à 20% dans une solution aqueuse de NaF à 625 ppm exprimés en fluorure F-. Le contact est maintenu 10 jours à 37°C sous agitation. La suspension est centrifugée. Le surnageant est dilué 20 fois à l'eau. La concentration en F⁻ est mesurée par ionométrie et comparée à la concentration de la solution initiale n'ayant eu aucun contact avec l'abrasif. On détermine ainsi la quantité de F⁻ n'ayant pas réagi. Le résultat de la compatibilité NaF est exprimé par

$$(F^- \text{ non réagi}/F^- \text{ de départ}) \times 100$$

Un deuxième objet de l'invention consiste en l'utilisation, comme agent abrasif ou additif dans les compositions dentifrices à base d'un composé fluoré, de particules composites comprenant un coeur de carbonate de calcium et une écorce de silice active susceptible d'être obtenues par précipitation sur du carbonate de calcium, de silice active à partir d'une solution aqueuse de silicate de métal alcalin M, de rapport $SiO_2/M_2O$ de 2 au moins, de préférence de 2,5 à 4, avec réglage du pH à l'aide d'un agent acidifiant, séparation de la bouillie de silice formée et séchage de la suspension de silice récupérée, lesdites particules contenant éventuellement à l'état adsorbé un agent stabilisant des ions $Ca^{2+}$. Les caractéristiques desdites particules, de même qu'un de leur mode performant de préparation ont déjà été mentionnés ci-dessus.

Elles peuvent être utilisées à raison de 5 à 40%, de préférence environ de 5 à 35% du poids desdites compositions.

Un dernier objet de l'invention consiste, à titre de produit industriel nouveau, en des particules composites constituées d'un coeur de carbonate de calcium et d'une écorce de silice active susceptible d'être obtenues par précipitation sur du carbonate de calcium, de silice active à partir d'une solution aqueuse de silicate de métal alcalin M, de rapport $SiO_2/M_2O$ de 2 au moins, de préférence de 2,5 à 4, avec réglage du pH à l'aide d'un agent acidifiant, séparation de la bouillie de silice formée et séchage de la suspension de silice récupérée, lesdites particules contenant à l'état adsorbé au moins un agent stabilisant des ions $Ca^{2+}$.

Parmi les agents stabilisants on peut citer des dérivés hydrosolubles du phosphore comme les phosphates de métaux alcalins (pyrophosphates, orthophosphates, tripolyphosphates, hexametaphosphates de sodium, potassium, lithium). La quantité dudit agent stabilisant peut représenter jusqu'à 2% de la masse des particules comprenant une écorce de silice active dense et un coeur de carbonate de calcium, exprimée en sec.

Un mode de préparation desdites particules composites a déjà été mentionné ci-dessus.

Les exemples suivants sont donnés à titre illustratif

**Exemple 1**

On prépare un pied de cuve, par introduction dans un réacteur de 15 litres, de 3,5 litres d'eau, de 0,68 mole/litre de pied de cuve, de sodium sous forme de chlorure de sodium, de 1150g de carbonate de calcium précipité (STURCAL H commercialisé par RHONE-POULENC, présentant une granulométrie de 11μm, une surface spécifique BET de 4 m²/g) et de silicate de sodium de rapport $SiO_2/Na_2O$ de 3,5 (solution aqueuse à 130g de $SiO_2$ par litre) en quantité correspondant à une concentration de 4g de $SiO_2$ par litre de pied de cuve. Le pied de cuve de pH 9 est porté à 90°C et maintenu sous agitation.

On introduit ensuite simultanément

- une solution aqueuse de silicate de sodium de rapport $SiO_2/Na_2O$ de 3,5 dont la concentration est de 130g de $SiO_2$ par litre de solution,
- et une solution aqueuse d'acide sulfurique contenant 80g d'acide par litre,
  de façon à former 230g de silice en 90 minutes.

Après mûrissement pendant 30 minutes, la bouillie obtenue est filtrée ; le gâteau de filtration est lavé à l'eau puis séché à l'étuve à 80°C.

L'analyse du produit par microscopie électronique (MET) montre que l'épaisseur de la couche de silice déposée est de l'ordre de 20nm.

La surface BET des particules finales est de 2,9 m²/g.

La cinétique d'ajout du silicate de sodium a été de 0,134g($SiO_2$)/h/g($CaCO_3$).

**Exemple 2**

L'abrasif préparé à l'exemple 1 est testé quant à sa compatibilité avec le MFP en solution dans l'eau et dans les mélanges sorbitol/eau et glycérol/eau.

Les résultats obtenus sont donnés au tableau 1 et comparés à ceux obtenus avec la même quantité de STURCAL H (carbonate de calcium précipité commercialisé par RHONE-POULENC, présentant une granulométrie de 11μm, une surface spécifique BET de 4 m²/g) comme agent abrasif.

### Exemple 3

L'abrasif préparé à l'exemple 1 est testé quant à sa compatibilité avec le MFP, au sein de la composition dentifrice ci-dessus décrite.
Les résultats obtenus sont donnés au tableau 1 et comparés à ceux obtenus avec du STURCAL H.

### Exemple 4

On prépare un pied de cuve, par introduction dans un réacteur de 15 litres de 3,5 litres d'eau, de 0,68mole/litre de pied de cuve, de sodium sous forme de chlorure de sodium, de 1150g de carbonate de calcium précipité (STURCAL H commercialisé par RHONE-POULENC, présentant une granulométrie de 11μm, une surface spécifique BET de 4 m$^2$/g) et de silicate de sodium de rapport $SiO_2/Na_2O$ de 3,5 (solution aqueuse à 130g de $SiO_2$ par litre) en quantité correspondant à une concentration de 4g de $SiO_2$ par litre de pied de cuve. Le pied de cuve de pH 9 est porté à 90°C et maintenu sous agitation. On introduit ensuite simultanément

- une solution aqueuse de silicate de sodium de rapport $SiO_2/Na_2O$ de 3,5 dont la concentration est de 130g de $SiO_2$ par litre de solution,
- et une solution aqueuse d'acide sulfurique contenant 80g d'acide par litre,
  de façon à former 460g de silice en 180 minutes.

Après mûrissement pendant 30 minutes, la bouillie obtenue est filtrée ; le gâteau de filtration est lavé à l'eau puis séché à l'étuve à 80°C.
L'analyse du produit par microscopie électronique (MET) montre que l'épaisseur de la couche de silice déposée est de l'ordre de 40nm.
La surface BET des particules finales est de 2,4 m$^2$/g.
La cinétique d'ajout du silicate de sodium a été de 0,134g($SiO_2$)/h/g($CaCO_3$).

### Exemple 5

L'abrasif préparé à l'exemple 4 est testé quant à sa compatibilité avec le NaF.
Les résultats obtenus sont donnés au tableau 2 et comparés à ceux obtenus avec la même quantité de STURCAL H (carbonate de calcium précipité commercialisé par RHONE-POULENC, présentant une granulométrie de 11μm, une surface spécifique BET de 4 m$^2$/g) comme agent abrasif.

Tableau 1

| milieu eau | compatibilité MFP en % | ppm de F⁻ avant hydrolyse |
|---|---|---|
| abrasif de l'exemple1 | 83 | 165 |
| STURCALH | 76 | 142 |

| milieu sorbitol/eau 50/50 | compatibilité MFP en % | ppm de F⁻ avant hydrolyse |
|---|---|---|
| abrasif de l'exemple1 | 69 | 113 |
| STURCAL H | 57 | 96 |

| milieu glycéro/eau 35/65 | compatibilité MFP en % | ppm de F' avant hydrolyse |
|---|---|---|
| abrasif de l'exemple1 | 83 | 126 |
| STURCAL H | 65 | 118 |

| milieu dentifrice | compatibilité MFP en % | ppm de F' avant hydrolyse |
|---|---|---|
| abrasif de l'exemple1 | 61 | 92 |
| STURCAL H | 40 | 165 |

Tableau 2

| milieu eau | compatibilité NaF en % |
|---|---|
| abrasif de l'exemple 4 | 88 |
| STURCAL H | 75 |

**Revendications**

1. Composition dentifrice comprenant au moins un agent pour la prévention des caries, à base d'un composé fluoré en une quantité correspondant à une concentration de 0,005 à 2%, de préférence de 0,1 à 1% en poids de fluor dans ladite composition, et au moins une silice, composition **caractérisée en ce que** ladite silice représente de 5 à 40% , de préférence de 5 à 35% du poids de ladite composition et est constituée de particules comprenant une écorce de silice active et un coeur de carbonate de calcium susceptibles d'être obtenues par précipitation sur du carbonate de calcium, de silice active à partir d'une solution aqueuse de silicate de métal alcalin M, de rapport $SiO_2/M_2O$ de 2 au moins, de préférence de 2,5 à 4, avec réglage du pH à l'aide d'un agent acidifiant, séparation de la bouillie de silice formée et séchage de la suspension de silice récupérée.

2. Composition dentifrice selon la revendication 1), **caractérisée en ce que** les particules de silice présentent une écorce de silice active d'épaisseur de 2 à 200 nm, de préférence de 5 à 50nm, pour une taille de coeur de carbonate de calcium de 20nm à 30µm, de préférence de 50 nm à 20 µm.

3. Composition dentifrice selon la revendication 1), **caractérisée en ce que** les particules de silice présentent une surface spécifique BET de 1 à 100 $m^2/g$, de préférence de 1 à 40 $m^2/g$.

4. Composition dentifrice selon l'une quelconque des revendications précédentes, **caractérisée en ce que** les particules de silice présentent une abrasivité RDA de 30 à 250, de préférence de 40 à 200.

5. Composition dentifrice selon l'une quelconque des revendications 1) à 4), **caractérisée en ce que** l'opération de formation de bouillie de silice par précipitation est réalisée selon les étapes suivantes :

   - une première étape consistant à mettre en oeuvre un pied de cuve initial de pH de 8 à 10, comprenant de l'eau, du carbonate de calcium, un sel électrolyte du groupe des métaux alcalins, la quantité d'électrolyte présente étant d'au moins 0,4 mole, de préférence de 0,4 à 1,5 mole d'ion métal alcalin par litre de pied de cuve, éventuellement un agent basique ou tampon, à une température de 80 à 98°C ;
   - une deuxième étape consistant à introduire dans ledit pied de cuve, le silicate de métal alcalin sous forme d'une solution aqueuse contenant au moins 100 grammes de $SiO_2$ / litre , de préférence de 100 à 330 grammes de $SiO_2$ / litre, et l'agent acidifiant, dans des conditions telles que la cinétique C de formation de silice active, exprimée en grammes de silice / heure / gramme de carbonate de calcium, corresponde à une valeur

$$C \geq 3 \, (A/200) \, 2^n,$$

de préférence $C \geq 4 \, (A/200) \, 2^n$
et tout particulièrement $C \geq 6 \, (A/200) \, 2^n$

   - $\underline{n}$ étant égal à $(T-90)/10$
   - $\underline{A}$ représentant la surface spécifique, exprimée en $m^2/g$, du carbonate à enrober
   - et T la température en °C,

le milieu réactionnel présentant un pH sensiblement constant de 8 à 10 et étant maintenu à une température de 80 à 98°C,
et ce jusqu'à ce que la quantité désirée de silice soit formée.

6. Composition dentifrice selon l'une quelconque des revendications 1) à 5), **caractérisée en ce que** le silicate de métal alcalin est un silicate de sodium ou de potassium.

**7.** Composition dentifrice selon l'une quelconque des revendication 1) à 6), **caractérisée en ce que** l'agent acidifiant est choisi parmi l'acide sulfurique, l'acide nitrique, l'acide chlorhydrique, l'acide acétique, l'acide formique ou l'acide carbonique.

**8.** Composition dentifrice selon la revendication 7), **caractérisée en ce que** l'agent acidifiant est de l'acide sulfurique.

**9.** Composition dentifrice selon l'une quelconque des revendication 5) à 8), **caractérisée en ce que** la quantité carbonate de calcium mise en oeuvre est telle que le pied de cuve formé contienne de 10% à 50% de son poids de carbonate de calcium.

**10.** Composition dentifrice selon l'une quelconque des revendication 1) à 9), **caractérisée en ce que** le carbonate de calcium mis en oeuvre présente une taille de 20nm à 30µm, de préférence de 50nm à 20µm.

**11.** Composition dentifrice selon l'une quelconque des revendication 5) à 10), **caractérisée en ce que** l'électrolyte est choisi parmi le sulfate de sodium, le chlorure de sodium, le nitrate de sodium ou l'hydrogénocarbonate de sodium.

**12.** Composition dentifrice selon l'une quelconque des revendication 1) à 11), **caractérisée en ce** le composé fluoré est un sel de l'acide monofluorophosphorique ou un fluorure de métal alcalin.

**13.** Composition dentifrice selon la revendication 12), **caractérisée en ce** le composé fluoré est un sel de sodium, potassium, lithium, calcium, aluminium ou ammonium de l'acide monofluorophosphorique ou du fluorure de sodium.

**14.** Composition dentifrice selon l'une quelconque des revendications 1) à 13), **caractérisée en ce que** l'écorce de silice dense des particules contient à l'état adsorbé au moins un agent stabilisant des ions $Ca^{2+}$.

**15.** Composition dentifrice selon la revendication 14), **caractérisée en ce que** l'agent stabilisant des ions $Ca^{2+}$ est un phosphate de métal alcalin, de préférence un pyrophosphate, orthophosphate, tripolyphosphate ou hexamétaphosphate de sodium, potassium, lithium.

**16.** Composition dentifrice selon la revendication 14) ou 15), **caractérisée en ce que** ledit agent stabilisant des ions $Ca^{2+}$ est adsorbé à la surface de l'écorce de silice par traitement de l'écorce de silice par une solution aqueuse dudit agent stabilisant, traitement réalisé par addition de ladite solution soit à la bouillie de silice formée à la fin de l'étape de précipitation, avant filtration, soit à la suspension de silice obtenue après filtration, avant séchage.

**17.** Composition dentifrice selon l'une quelconque des revendication 1) à 16), **caractérisée en ce qu'**elle contient en outre des agents tensio-actifs anioniques, non-ioniques, amphotères ou zwitterioniques, à raison de 0,1 à 10%, de préférence de 1 à 5% du poids de ladite composition, de l'eau à raison de 5 à 50%, de préférence 10 à 40% du poids de ladite composition, des agents humectants, à raison de 10 à 85%, de préférence de 10 à 70% du poids de ladite composition, des agents épaississants à raison de 0,1 à 15% en poids, éventuellement au moins un agent stabilisant des ions $Ca^{2+}$ et d'autres ingrédients choisis parmi les abrasifs polissants autres, les agents thérapeutiques bactéricides, anti-microbiens, anti-plaque, les agents arômatisants, les édulcorants, les colorants, les conservateurs.

**18.** Composition dentifrice selon l'une quelconque des revendication 1) à 17), **caractérisée en ce qu'**elle se présente sous forme de pâte, gel ou crème.

**19.** Utilisation, comme agent abrasif ou additif dans les compositions dentifrices à base d'un composé fluoré, de particules composites comprenant un coeur de carbonate de calcium et une écorce de silice active susceptibles d'être obtenues par précipitation sur du carbonate de calcium, de silice active à partir d'une solution aqueuse de silicate de métal alcalin M, de rapport $SiO_2/M_2O$ de 2 au moins, de préférence de 2,5 à 4, avec réglage du pH à l'aide d'un agent acidifiant, séparation de la bouillie de silice formée et séchage de la suspension de silice récupérée, lesdites particules contenant éventuellement à l'état adsorbé un agent stabilisant des ions $Ca^{2+}$.

**20.** Utilisation selon la revendication 19), **caractérisée en ce que** lesdites particules composites sont telles que définies à l'une quelconque des revendications 2) à 16).

**21.** Utilisation selon la revendication 19) ou 20), **caractérisée en ce que** lesdites particules composites sont mises en oeuvre à raison de 5 à 40%, de préférence 5 à 35% du poids desdites compositions dentifrices.

**22.** Particules composites constituées d'un coeur de carbonate de calcium et d'une écorce de silice active susceptibles d'être obtenues par précipitation sur du carbonate de calcium, de silice active à partir d'une solution aqueuse de silicate de métal alcalin M, de rapport $SiO_2/M_2O$ de 2 au moins, de préférence de 2,5 à 4, avec réglage du pH à l'aide d'un agent acidifiant, séparation de la bouillie de silice formée et séchage de la suspension de silice récupérée, lesdites particules contenant à l'état adsorbé au moins un agent stabilisant des ions $Ca^{2+}$.

**23.** Particules composites selon la revendication 22), **caractérisées en ce que** ledit stabilisant est un phosphate de métal alcalin, de préférence un pyrophosphate, orthophosphate, tripolyphosphate, hexametaphosphate de sodium, potassium ou lithium.

**24.** Particules composites selon la revendication 22) ou 23), **caractérisées en ce que** ledit stabilisant représenter jusqu'à 2% de la masse desdites particules composites.

**25.** Particules composites selon l'une quelconque des revendication 22) à 24), **caractérisées en ce que** ledit agent stabilisant des ions $Ca^{2+}$ est adsorbé à la surface de l'écorce de silice par traitement de l'écorce de silice par une solution aqueuse dudit agent stabilisant, traitement réalisé par addition de ladite solution soit à la bouillie de silice formée à la fin de l'étape de précipitation, avant filtration, soit à la suspension de silice obtenue après filtration, avant séchage.

**Patentansprüche**

**1.** Zahnpflegemittel-Zusammensetzung, die mindestens ein Mittel zur Prävention von Karies auf der Basis einer fluorierten Verbindung in einer Menge, die 0,005 bis 2 Gew.-%, vorzugsweise 0,1 bis 1 Gew.-% Fluor in der Zusammensetzung entspricht, und mindestens ein Siliciumdioxid umfaßt, wobei die Zusammensetzung **dadurch gekennzeichnet ist, daß** das Siliciumdioxid 5 bis 40 Gew.-%, vorzugsweise 5 bis 35 Gew.-% der Zusammensetzung ausmacht und aus Partikeln besteht, die eine Schale aus aktivem Siliciumdioxid und ein Herz aus Calciumcarbonat umfassen und die durch Präzipitation von aktivem Siliciumdioxid auf Calciumcarbonat, ausgehend von einer wäßrigen Lösung eines Silikats eines Alkalimetalls M mit einem $SiO_2/M_2O$-Verhältnis von mindestens 2, vorzugsweise 2,5 bis 4, unter Einstellung des pHs mit Hilfe eines acidifizierenden Mittels, Abtrennung der gebildeten Siliciumdioxid-Aufschlämmung und Trocknung der gewonnenen Siliciumdioxid-Suspension erhalten werden können.

**2.** Zahnpflegemittel-Zusammensetzung nach Anspruch 1, **dadurch gekennzeichnet, daß** die Siliciumdioxidpartikel eine Schale aus aktivem Siliciumdioxid einer Dicke von 2 bis 200 nm, vorzugsweise von 5 bis 50 nm bei einer Größe des Calciumcarbonatherzes von 20 nm bis 30 $\mu$m, vorzugsweise von 50 nm bis 20 $\mu$m, aufweisen.

**3.** Zahnpflegemittel-Zusammensetzung nach Anspruch 1, **dadurch gekennzeichnet, daß** die Siliciumdioxidpartikel eine spezifische Oberfläche nach BET von 1 bis 100 m$^2$/g, vorzugsweise von 1 bis 40 m$^2$/g, aufweisen.

**4.** Zahnpflegemittel-Zusammensetzung nach einem der vorangehenden Ansprüche, **dadurch gekennzeichnet, daß** die Siliciumdioxid-Partikel ein Abrasivvermögen RDA von 30 bis 250, vorzugsweise von 40 bis 200 haben.

**5.** Zahnpflegemittel-Zusammensetzung nach einem der Ansprüche 1 bis 4, **dadurch gekennzeichnet, daß** der Vorgang der Bildung der Siliciumdioxid-Aufschlämmung durch Präzipitation nach den folgenden Stufen durchgeführt wird:

- eine erste Stufe, die darin besteht, daß man einen Ansatz mit einem Anfangs-pH von 8 bis 10, der Wasser, Calciumcarbonat, ein Elektrolytsalz aus der Gruppe der Alkalimetalle, wobei die Elektrolytmenge, die vorliegt, mindestens 0,4 Mol, vorzugsweise 0,4 bis 1,5 Mol Alkalimetallion pro Liter Ansatz ist, gegebenenfalls ein basisches Mittel oder einen Puffer umfasst, bei einer Temperatur von 80 bis 98 °C herstellt;
- eine zweite Stufe, die darin besteht, in den Ansatz das Silikat eines Alkalimetalls in Form einer wäßrigen Lösung, die mindestens 100 g SiO$_2$/Liter, vorzugsweise 100 bis 330 g SiO$_2$/Liter und das acidifizierende Mittel enthält, unter solchen Bedingungen einzuführen, daß die Kinetik C der Bildung von aktivem Siliciumdioxid, ausgedrückt in Gramm Siliciumdioxid/Stunde/Gramm Calciumcarbonat, einem Wert

$$C \geq 3 \ (A/200)2^n,$$

vorzugsweise $C \geq 4 \ (A/200)2^n$
und besonders bevorzugt $C \geq 6 \ (A/(200)2^n$
entspricht,

- worin n gleich (T-90)/10 ist,
- A die spezifische Oberfläche, ausgedrückt in $m^2$/g des zu umhüllenden Carbonats ist und T die Temperatur in °C ist,

wobei das Reaktionsmilieu einen pH etwa konstant von 8 bis 10 aufweist und bei einer Temperatur von 80 bis 98 °C gehalten wird, bis sich die gewünschte Menge an Siliciumdioxid gebildet hat.

6. Zahnpflegemittel-Zusammensetzung nach einem der Ansprüche 1 bis 5, **dadurch gekennzeichnet, daß** das Silikat eines Alkalimetalls Natriumsilikat oder Kaliumsilikat ist.

7. Zahnpflegemittel-Zusammensetzung nach einem der Ansprüche 1 bis 6, **dadurch gekennzeichnet, daß** das acidifizierende Agens unter Schwefelsäure, Salpetersäure, Salzsäure, Essigsäure, Ameisensäure und Kohlensäure ausgewählt ist.

8. Zahnpflegemittel-Zusammensetzung nach Anspruch 7, **dadurch gekennzeichnet, daß** das acidifizierende Mittel Schwefelsäure ist.

9. Zahnpflegemittel-Zusammensetzung nach einem der Ansprüche 5 bis 8, **dadurch gekennzeichnet, daß** die eingesetzte Calciumcarbonatmenge so ist, daß der gebildete Ansatz Calciumcarbonat in einer Menge von 10 % bis 50 % seines Gewichts enthält.

10. Zahnpflegemittel-Zusammensetzung nach einem der Ansprüche 1 bis 9, **dadurch gekennzeichnet, daß** das eingesetzte Calciumcarbonat eine Größe von 20 nm bis 30 μm, vorzugsweise von 50 nm bis 20 μm aufweist.

11. Zahnpflegemittel-Zusammensetzung nach einem der Ansprüche 5 bis 10, **dadurch gekennzeichnet, daß** der Elektrolyt unter Natriumsulfat, Natriumchlorid, Natriumnitrat und Natriumhydrogencarbonat ausgewählt ist.

12. Zahnpflegemittel-Zusammensetzung nach einem der Ansprüche 1 bis 11, **dadurch gekennzeichnet, daß** die Fluorverbindung ein Salz der Monofluorphosphorsäure oder ein Alkalimetallfluorid ist.

13. Zahnpflegemittel-Zusammensetzung nach Anspruch 12, **dadurch gekennzeichnet, daß** die fluorierte Verbindung ein Natrium-, Kalium-, Lithium-, Calcium-, Aluminiumoder Ammonium-Salz der Monofluorphosphorsäure oder Natriumfluorid ist.

14. Zahnpflegemittel-Zusammensetzung nach einem der Ansprüche 1 bis 13, **dadurch gekennzeichnet, daß** der dichte Mantel aus Siliciumdioxid der Partikel mindestens ein Stabilisierungsmittel für $Ca^{2+}$-Ionen in adsorbiertem Zustand enthält.

15. Zahnpflegemittel-Zusammensetzung nach Anspruch 14, **dadurch gekennzeichnet, daß** das Stabilisierungsmittel für $Ca^{2+}$-Ionen ein Alkalimetallphosphat, vorzugsweise ein Pyrophosphat, Orthophosphat, Tripolyphosphat oder Hexametaphosphat von Natrium, Kalium, Lithium ist.

16. Zahnpflegemittel-Zusammensetzung nach Anspruch 14 oder 15, **dadurch gekennzeichnet, daß** das Stabilisierungsmittel für $Ca^{2+}$-Ionen durch Behandlung des Mantels aus Siliciumdioxid mit einer wäßrigen Lösung des Stabilisierungsmittels an der Oberfläche des Mantels aus Siliciumdioxid adsorbiert ist, wobei die Behandlung durch Zusatz der genannten Lösung entweder zu der gebildeten Siliciumdioxid-Aufschlämmung am Ende der Präzipitationsstufe vor der Filtration oder zur erhaltenen Siliciumdioxid-Suspension nach der Filtration, vor der Trocknung, durchgeführt wird.

17. Zahnpflegemittel-Zusammensetzung nach einem der Ansprüche 1 bis 16, **dadurch gekennzeichnet, daß** sie außerdem anionische, nichtionische, amphotere oder zwitterionische oberflächenaktive Mittel in einem Anteil von 0,1 bis 10 %, vorzugsweise von 1 bis 5 % des Gewichts der Zusammensetzung, Wasser in einem Anteil von 5 bis 50 %, vorzugsweise 10 bis 40 % des Gewichts der Zusammensetzung, Feuchthaltemittel in einem Anteil von 10 bis 85 %, vorzugsweise 10 bis 70 % des Gewichts der Zusammensetzung, Verdickungsmittel in einem Anteil von 0,1 bis 15 Gew.-%, gegebenenfalls mindestens ein Stabilisierungsmittel für $Ca^{2+}$-Ionen und andere Ingredienzien, ausgewählt aus anderen polierenden Schleifmitteln, therapeutischen bakteriziden Mitteln, antimikrobiellen Mitteln, Antiplaque-Mitteln, Aromastoffen, Süßstoffen, Färbemitteln, Konservierungsstoffen, enthält.

18. Zahnpflegemittel-Zusammensetzung nach einem der Ansprüche 1 bis 17, **dadurch gekennzeichnet, daß** sie sich in Form einer Paste, eines Gels oder einer Creme präsentiert.

19. Verwendung von Verbundpartikeln als Schleifmittel oder Additiv in den Zahnpflegemittel-Zusammensetzungen auf der Basis einer fluorierten Verbindung, wobei die Verbundpartikel ein Herz aus Calciumcarbonat und einen Mantel aus aktivem Siliciumdioxid umfassen und durch Präzipitation von aktivem Siliciumdioxid auf Calciumcarbonat, ausgehend von einer wäßrigen Lösung eines Silikats eines Alkalimetalls M mit einem $SiO_2/M_2O$-Verhältnis von mindestens 2, vorzugsweise 2,5 bis 4, bei Einstellung des pHs mit Hilfe eines acidifizierenden Mittels, Abtrennung der gebildeten Siliciumdioxid-Aufschlämmung und Trocknung der gewonnen Siliciumdioxid-Suspension, wobei die Partikel ein Stabilisierungsmittel für die $Ca^{2+}$-Ionen gegebenenfalls in adsorbiertem Zustand enthalten.

20. Verwendung nach Anspruch 19, **dadurch gekennzeichnet, daß** die Verbundpartikel so sind, wie sie in einem der Ansprüche 2 bis 16 definiert sind.

21. Verwendung nach Anspruch 19 oder 20, **dadurch gekennzeichnet, daß** die Verbundpartikel in einem Anteil von 5 bis 40 %, vorzugsweise von 5 bis 35 % des Gewichts der Zahnpflegemittel-Zusammensetzungen verwendet werden.

22. Verbundpartikel, gebildet aus einem Calciumcarbonatherz und einem Mantel aus aktivem Siliciumdioxid, die durch Präzipitation von aktivem Siliciumdioxid auf Calciumcarbonat, ausgehend'von einer wäßrigen Lösung eines Silikats eines Alkalimetalls M mit einem $SiO_2/M_2O$-Verhältnis von mindestens 2, vorzugsweise von 2,5 bis 4, bei Einstellung des pHs mit Hilfe eines acidifizierenden Mittels, Abtrennung der gebildeten Siliciumdioxid-Aufschlämmung und Trocknung der gewonnenen Siliciumdioxid-Suspension erhalten werden können, wobei die Partikel mindestens ein Stabilisierungsmittel für $Ca^{2+}$-Ionen in adsorbiertem Zustand enthalten.

23. Verbundpartikel nach Anspruch 22, **dadurch gekennzeichnet, daß** das Stabilisierungsmittel ein Alkalimetallphosphat, vorzugsweise ein Pyrophosphat, Orthophosphat, Tripolyphosphat, Hexametaphosphat von Natrium, Kalium oder Lithium ist.

24. Verbundpartikel nach Anspruch 22 oder 23, **dadurch gekennzeichnet, daß** das Stabilisierungsmittel bis zu 2 % der Masse der Verbundpartikel darstellt.

25. Verbundpartikel nach einem der Ansprüche 22 bis 24, **dadurch gekennzeichnet, daß** das Stabilisierungsmittel für die $Ca^{2+}$-Ionen durch Behandlung des Mantels aus Siliciumdioxid mit einer wäßrigen Lösung des Stabilisierungsmittels an der Oberfläche des Mantels aus Siliciumdioxid adsorbiert ist, wobei die Behandlung durch Zusatz der Lösung zu der gebildeten Siliciumdioxid-Aufschlämmung am Ende der Präzipitationsstufe vor der Filtration oder zu der erhaltenen Siliciumdioxid-Suspension nach der Filtration, vor der Trocknung, durchgeführt wird.

**Claims**

1. Dentifrice composition comprising at least one agent for the prevention of caries, which agent is based on a fluorinated compound in an amount corresponding to a concentration of 0.005 to 2%, preferably of 0.1 to 1%, by weight of fluorine in the said composition, and at least one silica, which composition is **characterized in that** the said silica represents from 5 to 40%, preferably from 5 to 35%, of the weight of the said composition and is composed of particles comprising an active silica shell and a calcium carbonate core which are capable of being obtained by precipitation on calcium carbonate of active silica from an aqueous alkali metal M silicate solution, with an $SiO_2/M_2O$ ratio of at least 2, preferably of 2.5 to 4, with adjustment of the pH using an acidifying agent, separation of the silica slurry formed and drying of the silica suspension recovered.

2. Dentifrice composition according to Claim 1, **characterized in that** the silica particles exhibit an active silica shell with a thickness of 2 to 200 nm, preferably of 5 to 50 nm, for a calcium carbonate core size of 20 nm to 30 μm,

preferably of 50 nm to 20 μm.

3. Dentifrice composition according to Claim 1, **characterized in that** the silica particles exhibit a BET specific surface of 1 to 100 $m^2$/g, preferably of 1 to 40 $m^2$/g.

4. Dentifrice composition according to any one of the preceding claims, **characterized in that** the silica particles exhibit an RDA abrasiveness of 30 to 250, preferably of 40 to 200.

5. Dentifrice composition according to any one of Claims 1 to 4, **characterized in that** the operation of formation of silica slurry by precipitation is carried out according to the following stages:

   - a first stage consisting in employing an initial vessel heel with a pH of 8 to 10 comprising water, calcium carbonate, an electrolyte salt from the group of alkali metals, the amount of electrolyte present being at least 0.4 mol, preferably from 0.4 to 1.5 mol, of alkali metal ion per litre of vessel heel, and optionally a buffer or basic agent, at a temperature of 80 to 98°C;
   - a second stage consisting in introducing, into the said vessel heel, the alkali metal silicate in the form of an aqueous solution containing at least 100 grams of $SiO_2$/litre, preferably from 100 to 330 grams of $SiO_2$/litre, and the acidifying agent, under conditions such that the kinetics K of formation of active silica, expressed in grams of silica/hour/gram of calcium carbonate, corresponds to a value

$$K \geq 3 \, (A/200) \, 2^n,$$

   preferably $K \geq 4 \, (A/200) \, 2^n$
   and very particularly $K \geq 6 \, (A/200) \, 2^n$

   - $\underline{n}$ being equal to $(T-90)/10$
   - $\underline{A}$ representing the specific surface, expressed in $m^2$/g, of the carbonate to be coated
   - and T the temperature in °C,

   the reaction mixture exhibiting a substantially constant pH of 8 to 10 and being maintained at a temperature of 80 to 98°C,
   until the desired amount of silica has been formed.

6. Dentifrice composition according to any one of Claims 1 to 5, **characterized in that** the alkali metal silicate is a sodium or potassium silicate.

7. Dentifrice composition according to any one of Claims 1 to 6, **characterized in that** the acidifying agent is chosen from sulphuric acid, nitric acid, hydrochloric acid, acetic acid, formic acid or carbonic acid.

8. Dentifrice composition according to Claim 7, **characterized in that** the acidifying agent is sulphuric acid.

9. Dentifrice composition according to any one of Claims 5 to 8, **characterized in that** the amount of calcium carbonate employed is such that the vessel heel formed contains from 10% to 50% of its weight of calcium carbonate.

10. Dentifrice composition according to any one of Claims 1 to 9, **characterized in that** the calcium carbonate employed exhibits a size of 20 nm to 30 μm, preferably of 50 nm to 20 μm.

11. Dentifrice composition according to any one of Claims 5 to 10, **characterized in that** the electrolyte is chosen from sodium sulphate, sodium chloride, sodium nitrate or sodium hydrogencarbonate.

12. Dentifrice composition according to any one of Claims 1 to 11, **characterized in that** the fluorinated compound is a salt of monofluorophosphoric acid or an alkali metal fluoride.

13. Dentifrice composition according to Claim 12, **characterized in that** the fluorinated compound is a sodium, potassium, lithium, calcium, aluminium or ammonium salt of monofluorophosphoric acid or sodium fluoride.

14. Dentifrice composition according to any one of Claims 1 to 13, **characterized in that** the dense silica shell of the particles contains, in the adsorbed state, at least one agent for stabilizing $Ca^{2+}$ ions.

**15.** Dentifrice composition according to Claim 14, **characterized in that** the agent for stabilizing $Ca^{2+}$ ions is an alkali metal phosphate, preferably a sodium, potassium or lithium pyrophosphate, orthophosphate, tripolyphosphate or hexametaphosphate.

**16.** Dentifrice composition according to Claim 14 or 15, **characterized in that** the said agent for stabilizing $Ca^{2+}$ ions is adsorbed at the surface of the silica shell by treating the silica shell with an aqueous solution of the said stabilizing agent, which treatment is carried out by addition of the said solution either to the silica slurry formed at the end of the precipitation stage, before filtration, or to the silica suspension obtained after filtration, before drying.

**17.** Dentifrice composition according to any one of Claims 1 to 16, **characterized in that** it additionally contains anionic, non-ionic, amphoteric or zwitterionic surface-active agents in the proportion of 0.1 to 10%, preferably of 1 to 5%, of the weight of the said composition, water in the proportion of 5 to 50%, preferably 10 to 40%, of the weight of the said composition, humectants in the proportion of 10 to 85%, preferably of 10 to 70%, of the weight of the said composition, thickening agents in the proportion of 0.1 to 15% by weight, optionally at least one agent for stabilizing $Ca^{2+}$ ions and other ingredients chosen from other polishing abrasives, bactericidal, antimicrobial or anti-plaque therapeutic agents, flavourings, sweeteners, colourings or preservatives.

**18.** Dentifrice composition according to any one of Claims 1 to 17, **characterized in that** it is provided in the paste, gel or cream form.

**19.** Use, as additive or abrasive agent in dentifrice compositions based on a fluorinated compound, of composite particles comprising a calcium carbonate core and an active silica shell which are capable of being obtained by precipitation on calcium carbonate of active silica from an aqueous alkali metal M silicate solution, with an $SiO_2/M_2O$ ratio of at least 2, preferably of 2.5 to 4, with adjustment of the pH using an acidifying agent, separation of the silica slurry formed and drying of the silica suspension recovered, the said particles optionally containing, in the adsorbed state, an agent for stabilizing $Ca^{2+}$ ions.

**20.** Use according to Claim 19, **characterized in that** the said composite particles are as defined in any one of Claims 2 to 16.

**21.** Use according to Claim 19 or 20, **characterized in that** the said composite particles are employed in the proportion of 5 to 40%, preferably 5 to 35%, of the weight of the said dentifrice compositions.

**22.** Composite particles composed of a calcium carbonate core and of an active silica shell which are capable of being obtained by precipitation on calcium carbonate of active silica from an aqueous alkali metal M silicate solution, with an $SiO_2/M_2O$ ratio of at least 2, preferably of 2.5 to 4, with adjustment of the pH using an acidifying agent, separation of the silica slurry formed and drying of the silica suspension recovered, the said particles containing, in the adsorbed state, at least one agent for stabilizing $Ca^{2+}$ ions.

**23.** Composite particles according to Claim 22, **characterized in that** the said stabilizer is an alkali metal phosphate, preferably a sodium, potassium or lithium pyrophosphate, orthophosphate, tripolyphosphate or hexametaphosphate.

**24.** Composite particles according to Claim 22 or 23, **characterized in that** the said stabilizer represents up to 2% of the mass of the said composite particles.

**25.** Composite particles according to any one of Claims 22 to 24, **characterized in that** the said agent for stabilizing $Ca^{2+}$ ions is adsorbed at the surface of the silica shell by treating the silica shell with an aqueous solution of the said stabilizing agent, which treatment is carried out by addition of the said solution either to the silica slurry formed at the end of the precipitation stage, before filtration, or to the silica suspension obtained after filtration, before drying.